(19) Europäisches Patentamt
European Patent Office
Office européen des brevets

(11) Veröffentlichungsnummer: **0 004 561**
**B1**

(12) # EUROPÄISCHE PATENTSCHRIFT

(45) Veröffentlichungstag der Patentschrift: **04.11.81**

(21) Anmeldenummer: **79100647.1**

(22) Anmeldetag: **05.03.79**

(51) Int. Cl.³: **C 07 D 233/24,** C 07 D 233/06, C 07 D 239/06, C 07 D 239/14, C 07 D 403/12, A 61 K 31/415, A 61 K 31/44, A 61 K 31/505

(54) **Harnstoff- und Amidoverbindungen, deren Herstellung, pharmazeutische Präparate enthaltend diese Verbindungen und deren Verwendung zur Zubereitung von pharmazeutischen Präparaten.**

(30) Priorität: **08.03.78 CH 2519/78**

(43) Veröffentlichungstag der Anmeldung: **17.10.79 Patentblatt 79/21**

(45) Bekanntmachung des Hinweises auf die Patenterteilung: **04.11.81 Patentblatt 81/44**

(84) Benannte Vertragsstaaten: **BE CH DE FR GB IT LU NL SE**

(56) Entgegenhaltungen:
**CH-A-234 990**
**CH-A-485 843**
**FR-A-1 549 283**
**GB-A-813 525**

(73) Patentinhaber: **CIBA-GEIGY AG, Patentabteilung Postfach, CH-4002 Basel (CH)**

(72) Erfinder: **Marxer, Adrian, Prof. Dr., Rieserstrasse 4, CH-4132 Muttenz (CH)**

(74) Vertreter: **Zumstein sen., Fritz, Dr. et al, Bräuhausstrasse 4, D-8000 München 2 (DE)**

EP 0 004 561 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Harnstoff- und Amidoverbindungen, deren Herstellung, pharmazeutische Präparate enthaltend diese Verbindungen und deren Verwendung zur Zubereitung von pharmazeutischen Präparaten

Die vorliegende Erfindung betrifft neue Harnstoff- und Amidoverbindungen, deren Herstellung sowie pharmazeutische Präparate, die diese neuen Verbindungen enthalten, wie auch ihre Verwendung.

Im französischen Patent No. 1 549 283 werden im Phenylkern durch Halogen, Trifluormethyl, Cyano, Niederalkyl- oder Alkoxy-substituierte und im Imidazolinrest unsubstituierte 2-Anilino-methyl-imidazoline beschrieben, die blutdrucksenkende und vasokonstriktorische Eigenschaften aufweisen, wogegen die durch einen Harnstoff- bzw. Amidorest im Imidazolin- oder Tetrahydropyrimidinring substituierten erfindungsgemäßen Verbindungen starke Wirkungen auf Tumore haben.

Die Erfindung betrifft insbesondere neue Harnstoff- und Amidoverbindungen der Formel

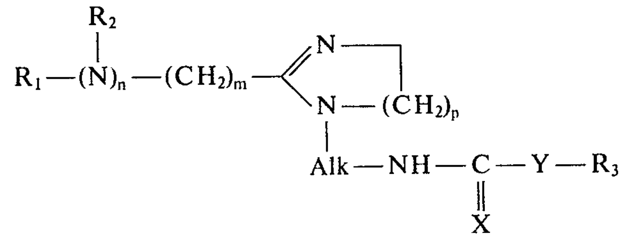

(I)

in der $R_1$ und $R_3$ ein monocyclisches, carbocyclisches Aryl, oder Heteroaryl, n 0 oder 1, m 0, 1 oder 2, p 1 oder 2, $R_2$ ein Wasserstoffatom oder Niederalkyl, Alk eine Niederalkylengruppe mit 2—3 Kohlenstoffatomen in der Kettenlinie, X Sauerstoff oder Schwefel bedeutet und Y die Iminogruppe oder eine direkte Bindung, und deren Salze ist.

Niedere Reste vor- und nachstehend sind vor allem solche Reste, die bis zu 7 C-Atome, insbesondere bis zu 4 C-Atome enthalten.

Ein monocyclischer, carbocyclischer Arylrest ist ein gegebenenfalls substituierter Phenylrest, beispielsweise ein einfach, zweifach oder auch mehrfach substituierter Phenylrest oder auch ein unsubstituierter Phenylrest.

Ein Heteroarylrest ist in erster Linie ein gegebenenfalls substituierter monoazacyclischer oder diazacyclischer 6gliedriger Rest oder monothiacyclischer Rest aromatischen Charakters, welcher z. B. gleich wie der monocyclischer, carbocyclischer Arylrest beispielsweise einfach, zweifach oder mehrfach substiuiert sein kann. Ein Heteroarylrest ist ein gegebenenfalls substiuierter Pyridyl-, Pyrimidinyl- oder Thienylrest, beispielsweise ein einfach, zweifach oder auch mehrfach substituierter Pyridyl-2-, Pyridyl-4-, Pyrimidinyl-2- oder Pyrimidinyl-4- oder Thienyl-2- oder Thienyl-3-rest.

Niederalkylreste sind beispielsweise Methyl. Äthyl, n-Propyl oder Isopropyl, oder geradkettiges oder verzweigtes Butyl, Pentyl, Hexyl oder Heptyl, die in beliebiger Stellung gebunden sein können.

Niederalkylen ist ein verzweigtes oder insbesondere geradkettiges Niederalkylen mit 2—3 C-Atomen in der Alkylenkette, wie 1,2-Propylen, 1,3-Propylen, 1,2-Butylen, 2,3-Butylen, 1,3-Butylen oder insbesondere 1,2-Äthylen.

Sind $R_1$ oder $R_3$ als Phenylrest oder als Pyridyl-, Pyrimidinyl-, Thienylrest ein- oder mehrfach substituiert, so können die Substituenten beispielsweise Niederalkyl, Halogen, Trifluormethyl, Niederalkoxy, Carboxyl oder Nieder-alkoxycarbonyl sein.

Ein Phenyl-, Pyridyl-, Pyrimidinyl- oder auch Thienylrest kann beispielsweise durch die oben definierten Niederalkylgruppen oder durch die weiter unten definierten Niederalkoxy-, Niederalkoxycarbonylgruppen, Halogen oder durch die Trifluormethylgruppe bzw. Carboxylgruppe substituiert sein.

Als Halogenatom kommen Halogenatome bis zur Atomnummer 35, wie z. B. Fluor, Chlor oder Brom in Betracht.

Niederalkoxy ist z. B. Methoxy, Äthoxy, n-Propyloxy, Isopropyloxy, n-Butyloxy oder n-Pentyloxy oder n-Hexyloxy. Niederalkoxycarbonyl ist beispielsweise Methoxy-, Äthoxy-, n-Propoxy-, Isopropyloxycarbonyl, oder geradkettige oder verzweigtes Butyloxy-, Pentyloxy-, Hexyloxy- oder Heptyloxy-carbonyl, die im Niederalkylrest in beliebiger Stellung gebunden sein können. Die neuen Verbindungen besitzen wertvolle pharmakologische Eigenschaften, insbesondere zeigen sie eine starke Wirkung bei Tumoren, z. B. bei durch Diäthylnitrosamin (DAENA)-induzierten epidermoiden Karzinomen der Lunge, der Trachea und des Kehlkopfes beim syrischen Goldhamster oder auch beim Erlich-Ascites-Karzinom der Maus.

So bewirkte die Behandlung von Goldhamstern mit respiratorischen Karzinomen (ab. 10. Wochen nach DAENA) mit Verbindungen wie z. B. dem 1-[2-[2-(2-Chlorphenyl)-2-imidazolin-1-yl]-äthyl]-3-(p-tolyl)-harnstoff oder dem 1-[2-[2-(4-Pyridyl)-2-imidazolin-1-yl]-äthyl]-3-(4-carboxy-phenyl)-harnstoff in Dosen von 12,5—100 mg/kg p. o., 5 Applikationen pro Woche 4 Wochen lang im Vergleich zu Kontrollen eine Dosis-abhängige Reduktion der Zahl der epidermoiden Karzinome in der Lunge bis zu 90%. Die Behandlung mit z. B. 50 mg/kg p. o. zeigte einen hoch signifikanten Unterschied ($P \leq 0.001$) zu

den Kontrollen. Die Hemmung des Tumorwachstums in Kehlkopf und Trachea betrug 65 bzw. 66%. Die Verbindungen sind daher bei Bronchialkarzinomen, die durch jetzige Zytostatika nicht beeinflußt werden, therapeutisch besonders wertvoll. Das Erlich-Ascites-Karzinom wird von einigen dieser Verbindungen, wie z. B. von dem 1-[2-[2-(Chloranilinomethyl)-2-imidazolin-1-yl]-äthyl)-3-(p-tolyl)-harnstoff stark gehemmt. Die Verträglichkeit ist gut. Nebenwirkungen oder makroskopisch sichtbare Organveränderungen werden auch nach 4 Wochen langer Behandlung nicht festgestellt. Ebenfalls findet sich keine kumulative Toxizität bei täglich wiederholter Behandlung im Vergleich zur einmaligen Verabreichung.

Die Erfindung betrifft in erster Linie Verbindungen der Formel I, in der $R_1$ und $R_3$ ein monocyclisches, carboxycyclisches Aryl oder Heteroaryl, n 0 oder 1, m 0, 1 oder 2, p 1 oder 2 bedeuten, mit der Maßgabe, daß, wenn m 0 bedeutet, n für 0 steht, $R_2$ ein Wasserstoffatom oder Niederalkyl, Alk eine Niederalkylengruppe mit 2—3 Kohlenstoffatomen in der Kettenlinie, X Sauerstoff oder Schwefel und Y die Iminogruppe oder eine direkte Bindung bedeuten und deren Salze.

Insbesondere betrifft die Erfindung diejenigen Verbindungen der Formel I, in der $R_1$ und $R_3$ ein gegebenenfalls substituiertes Phenyl oder Pyridyl, N 0 oder 1, m 0, 1 oder 2, p 1 bedeuten, mit der Maßgabe, daß wenn m 0 bedeutet, n für 0 steht, $R_2$ Wasserstoffatom, Alk eine Niederalkylengruppe mit 2 Kohlenstoffatomen in der Kettenlinie, X Sauerstoff oder Schwefel und Y die Iminogruppe oder eine direkte Bindung bedeuten und deren Salze.

Von besonderem Interesse sind Verbindungen der Formel I, in der $R_1$ und $R_3$ ein gegebenenfalls durch Halogen, Niederalkyl, Carboxyl oder Trifluormethyl substituiertes Phenyl oder Pyridyl, n 0 oder 1, m 0 oder 1, p 1 bedeuten, mit der Maßgabe, daß, wenn m 0 bedeutet, n für 0 steht, $R_2$ ein Wasserstoffatom, Alk eine Niederalkylengruppe mit 2 Kohlenstoffatomen in der Kettenlinie, X Sauerstoff und Y die Iminogruppe oder eine direkte Bindung bedeuten und deren Salze.

Von ganz besonderem Interesse sind Verbindungen der Formel I, in der $R_1$ ein gegebenenfalls durch Chlor, Methyl, Carboxyl oder Trifluormethyl substituiertes Phenyl oder Pyridyl-2 oder -4-, n 0 oder 1, m 0 oder 1, p 1 bedeuten, mit der Maßgabe, daß, wenn m 0 bedeutet, n für 0 steht, $R_2$ ein Wasserstoffatom, Alk eine 1,2-Äthylengruppe, X Sauerstoff und Y die Iminogruppe bedeuten und deren Salze.

Besonders zu nennen von diesen neuen Verbindungen der Formel I sind die in den Beispielen beschriebenen Verbindungen.

Die Verbindungen der allgemeinen Formel I werden hergestellt, indem man Verbindungen der allgemeinen Formel II,

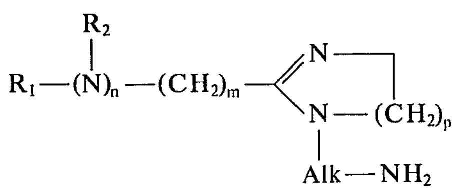

(II)

in der $R_1$, $R_2$, n, m, p und Alk die oben erwähnten Bedeutungen haben, mit einem reaktionsfähigen Derivat der Säure der allgemeinen Formel III

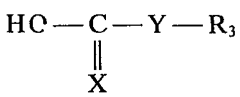

(III)

in der X, Y und $R_3$ die oben angegebene Bedeutungen haben, umsetzt und eine erhaltene Verbindung der allgemeinen Formel I gewünschtenfalls in ein Säureadditionssalz umwandelt.

Als reaktionsfähige Derivate der Säure der allgemeinen Formel III, in der Y eine Iminogruppe bedeutet, können innere Anhydride, d. h. Isocyanate bzw. Thioisocyanate der allgemeinen Formel IV

$$X{=}C{=}N{-}R_3 \qquad (IV)$$

in der X und $R_3$ die oben angegebenen Bedeutungen haben, für die Umsetzung verwendet werden. Die Umsetzungen werden in einem Temperaturbereich zwischen 0° und 140°C, vorzugsweise jedoch bei einer Temperatur zwischen 20° und 90°C in Gegenwart eines Lösungsmittels, wie z. B. einem niederen Alkanol oder Ester, beispielsweise Äthylazetat, einem Äther, wie z. B. Tetrahydrofuran, einem Keton wie z. B. Äthylmethylketon oder einem aromatischen Kohlenwasserstoff, wie z. B. Benzol, Toluol oder Xylol ausgeführt.

Als weitere reaktionsfähige Derivate der Säure der allgemeinen Formel III können Halogenide der Carbaminsäure bzw. Thiocarbaminsäure der allgemeinen Formel V

$$\mathrm{Hal-\underset{\underset{X}{\|}}{C}-NH-R_3} \qquad (V)$$

in der X und $R_3$ die oben angegebenen Bedeutungen haben, und Hal ein Halogenatom bedeutet, für die Umsetzung verwendet werden. Die Umsetzungen können in analoger Weise wie bei den Isocyanaten und Thioisocyanaten im gleichen Temperaturbereich und unter Verwendung eines analogen Lösungsmittels, in An- oder Abwesenheit von einem Trialkylamin, beispielsweise Triäthylamin, $K_2CO_3$ und dergleichen als säurebindendes Mittel, ausgeführt werden.

Als weitere reaktionsfähige Derivate der Säure der allgemeinen Formel III können Halogenide von $R_3$-Carbonsäuren der allgemeinen Formel Va

$$\mathrm{Hal-\underset{\underset{O}{\|}}{C}-R_3} \qquad (Va)$$

in der $R_3$ die oben gegebene Bedeutung hat, verwendet werden.

Die als Ausgangsstoffe verwendeten Verbindungen der Formel II sind bekannt und können durch Umsetzung eines gegebenenfalls substituierten Anilinomethylnitrils, Arylmethylnitrils oder Arylnitrils bzw. die entsprechenden Heteroarylverbindungen der Formel VI

$$\mathrm{R_1-(\overset{\overset{R_2}{|}}{N})_n-(CH_2)_m-CN} \qquad (VI)$$

mit dem entsprechenden Diäthylen- bzw. Dipropylentriamin (A. Marxer, J. A. Chem. Soc. 79, 467 (1957) erhalten werden.

Die verwendeten, gegebenenfalls substituierten Anilinomethylnitrile bzw. die entsprechenden Heteroarylverbindungen der Formel VI sind ebenfalls bekannt und werden durch Umsetzung aus den entsprechenden Anilinverbindungen bzw. Heteroarylverbindungen mit Formaldehyd und Cyanwasserstoff (A. Marxer, Helv. Chim. Acta 37, 166 (1954) erhalten.

In gleicher Weise wie die Ausgangsprodukte der allgemeinen Formel II können auch Verbindungen der allgemeinen Formel I hergestellt werden, in der $R_1$, $R_2$, $R_3$, X, Y, n, m und p die oben angegebenen Bedeutungen haben, indem man eine Verbindung der allgemeinen Formel VI

$$\mathrm{R_1-(\overset{\overset{R_2}{|}}{N})_n-(CH_2)_m-CN} \qquad (VI)$$

mit einem Diäthylen- bzw. Dipropylentriaminderivat der allgemeinen Formel VII

$$\mathrm{H_2N-CH_2(CH_2)_p-HN-Alk-NH-\underset{\underset{X}{\|}}{C}-Y-R_3} \qquad (VII)$$

(A. Marxer, J. A. Chem. Soc. 79, 467 (1957)) umsetzt.

Verbindungen der allgemeinen Formel I, in der $R_3$ durch eine Niederalkoxycarbonylgruppe, wie z. B. durch eine Äthoxycarbonylgruppe substituiert ist, können durch Verseifung, d. h. durch eine Hydrolyse in saurem oder alkalischem Medium, in Verbindungen der Formel I übergeführt werden, in der $R_3$ z. B. erinen durch einen Carboxylgruppe substituierten Phenyl- oder Pyridylrest bedeutet. Die Hydrolyse wird vorzugsweise in alkalischem Medium, d. h. in Gegenwart einer starken Alkali- oder Erdalkalibase, wie z. B. Natronlauge, ausgeführt.

Je nach den Verfahrensbedingungen und Ausgangsstoffen erhält man die Endstoffe in freier Form oder in der ebenfalls in der Erfindung inbegriffenen Form ihrer Säureadditionssalze. So können beispielsweise basische, neutrale oder gemischte Salze, gegebenenfalls auch Hemi-, Mono-, Sesqui- oder Polyhydrate davon erhalten werden. Die Säureadditionssalze der neuen Verbindungen können in an sich bekannter Weise in die freie Verbindung übergeführt werden, z. B. mit basischen Mitteln, wie Alkalien oder Ionenaustauschern. Andererseits können die erhaltenen freien Basen mit organischen oder anorganischen Säuren Salze bilden. Zur Herstellung von Säureadditionssalzen werden insbesondere solche Säuren verwendet, die zur Bildung von therapeutisch verwendbaren Salzen geeignet sind. Als solche Säuren seien beispielsweise genannt: Halogenwasserstoffsäuren, Schwefelsäure, Phosphorsäuren, Salpetersäure, aliphatische, alicyclische, aromatische oder heterocyclische Carbon- oder Sulfonsäuren, wie Ameisen-, Essig-, Propion-, Bernstein-, Glykol-, Milch-, Äpfel-, Wein-, Zitronen-, Ascorbin-, Malein-, Hydroxymalein- oder Benztraubensäure; Phenylessig-, Benzoe-, p-Aminobenzoe-, Anthranil-, p-Hydroxybenzoe-, Salicyl- oder p-Aminosalicylsäure, Embonsäure, Methansulfon-, Äthansulfon-, Hydroxyäthansulfon-, Äthylsulfonsäure; Halogenbenzolsulfon-, Toluolsulfon-, Naphthalinsulfonsäure oder Sulfanilsäure; Methionin, Trypthophan, Lysin oder Arginin.

Diese oder andere Salze der neuen Verbindungen, wie z. B. die Pikrate, können auch zur Reinigung der erhaltenen freien Basen dienen, indem man die freien Basen in Salze überführt, diese abtrennt und aus den Salzen widerum die Basen freimacht. Infolge der engen Beziehungen zwischen den neuen Verbindungen in freier Form und in Form ihrer Salze sind im Vorausgegangenen und nachfolgend unter den freien Verbindungen sinn- und zweckmäßig, gegebenenfalls auch die entsprechenden Salze zu verstehen.

Je nach der Zahl der asymmetrischen C-Atome und der Wahl der Ausgangsstoffe und Arbeitsweisen können die neuen Verbindungen als Racematgemische, als Racemate oder als optische Antipoden vorliegen.

Racematgemische können auf Grund der physikalisch-chemischen Unterschiede der Bestandteile in bekannter Weise in die reinen Racemate aufgetrennt werden, z. B. durch Chromatographie und/oder fraktionierte Kristallisation.

Reine Racemate lassen sich nach bekannten Methoden, beispielsweise durch Umkristallisation aus einem optisch aktiven Lösungsmittel, mit Hilfe von Mikroorganismen, oder durch Umsetzen mit einer, mit der racemischen Verbindung Salze bildenden optisch aktiven Säure und Trennung der auf diese Weise erhaltenen Salze, z. B. auf Grund ihrer verschiedenen Löslichkeit, in die Diastereomeren, aus denen die Antipoden durch Einwirkung geeigneter Mittel freigesetzt werden können, zerlegen. Besonders gebräuchliche optisch aktive Säuren sind z. B. die D- und L-Formen von Weinsäure, Di-o-Toluylweinsäure, Apfelsäure, Mandelsäure, Camphersulfonsäure oder Chinasäure. Vorteilhaft isoliert man den wirksameren der beiden Antipoden.

Erfindungsgemäß kann man aber auch die Endprodukte in Form der reinen Racemate bzw. optischen Antipoden erhalten, indem man ein oder mehrere asymmetrische C-Atome enthaltende Ausgangsstoffe in Form der reinen Racemate bzw. optischen Antipoden einsetzt.

Die neuen Wirkstoffe oder die pharmazeutisch annehmbaren Salze können enteral, wie oral oder rektal, sowie parenteral appliziert werden.

Die erfindungsgemäßen pharmazeutischen Stoffzusammensetzungen enthalten mindestens eine Verbindung der allgemeinen Formel I (s. oben) als Wirkstoff zusammen mit einem üblichen pharmazeutischen Trägerstoff. Die Art der Trägerstoffe richtet sich weitgehend nach dem Anwendungsgebiet.

Zur oralen Behandlung von Tumoren kommen insbesondere feste Doseneinheitsformen, wie Tabletten, Dragées und Kapseln in Frage. Die täglichen Dosen liegen zwischen 8 und 100 mg/kg Warmblüter. Geeignete Doseneinheitsformen wie Dragées oder Tabletten enthalten vorzugsweise 10–200 mg eines erfindungsgemäßen Wirkstoffes, wobei der Wirkstoffgehalt 10–90 Gewichtsprozent beträgt. Zur Herstellung von Tabletten und Dragées-Kernen kombiniert man die Verbindungen der allgemeinen Formel I mit festen, pulverförmigen Trägerstoffen, wie Lactose, Saccharose, Sorbit, Maisstärke, Kartoffelstärke oder Amylopektin, Cellulosederivaten oder Gelatine, vorzugsweise unter Zusatz von Gleitmitteln, wie Magnesium- oder Calciumstearat oder Polyäthylenglykolen von geeignetem Molekulargewicht. Dragée-Kerne überzieht man anschließend beispielsweise mit konzentrierten Zuckerlösungen, welche z. B. noch arabischen Gummi, Talk und/oder Titandioxd enthalten können, oder mit einem in leicht flüchtigen organischen Lösungsmittelgemischen gelösten Lack. Diesen Überzügen können Farbstoffe zugefügt werden, z. B. zur Kennzeichnung verschiedener Wirkstoffdosen. Weiche Gelatinekapseln und andere geschlossene Kapseln bestehen beispielsweise aus einem Gemisch von Gelatine und Glycerin und können z. B. Mischungen einer Verbindung der Formel I mit Polyäthylenglykol enthalten. Steckkapseln enthalten z. B. Granulate eines Wirkstoffes mit festen, pulverförmigen Trägerstoffen, wie z. B. Lactose, Saccharose, Sorbit, Mannit; Stärken, wie Kartoffelstärke, Maisstärke oder Amyloektin, Cellulosederivate und Gelatine sowie Magnesiumstearat oder Stearinsäure.

Die nachfolgenden Beispiele a) bis d) sollen die Herstellung einiger typischer Applikationsformen erläutern, jedoch keineswegs die einzigen Ausführungsformen von solchen darstellen.

a) 250,0 g Wirkstoff werden mit 550,0 g Lactose und 292,0 g Kartoffelstärke vermischt, die Mischung mit einer alkoholischen Lösung von 8 g Gelatine befeuchtet und durch ein Sieb granuliert. Nach dem Trocknen mischt man 60,0 g Kartoffelstärke, 60,0 g Talk, 10,0 g Magnesiumstearat und 20,0 g kolloidales Siliciumdioxid zu und preßt die Mischung zu 10 000 Tabletten von je 125 mg Gewicht und 25 mg Wirkstoffgehalt, die gewünschtenfalls mit Teilkerben zur feineren Anpassung der Dosierung versehen sein können.

b) Aus 100,0 g Wirkstoff, 379,0 g Lactose und der der alkoholischen Lösung von 6,0 g Gelatine stellt man ein Granulat her, das man nach dem Trocknen mit 10,0 g Kolloidalem Siliciumdioxid, 40,0 g Talk, 60,0 g Kartoffelstärke und 5,0 g Magnesiumstearat mischt und zu 10 000 Dragée-Kernen preßt. Diese werden anschließend mit einem konzentrierten Sirup aus 533,5 g krist. Sachharose, 20,0 g Schellack, 75,0 g arabischem Gummi, 250,0 g Talk, 20,0 g kolloidalem Siliciumdioxid und 1,5 g Farbstoff überzogen und getrocknet. Die erhaltenen Dragées wiegen je 150 mg und

enthalten je 10 mg Wirkstoff.

c) Zur Bereitung eines Sirups mit 0,25% Wirkstoffgehalt löst man in 3 Litern dest. Wasser 1,5 Liter Glycerin, 42 g p-Hydroxybenzoesäure-methylester, 18 g p-Hydroxybenzoesäure-n-propylester und unter leichtem Erwärmen 25,0 g Wirkstoff fügt man 4 Liter 70%ige Sorbitlösung, 1000 g krist. Saccharose, 350 g Glucose und einen Aromastoff, z. B. 250 g »Orange Peel Soluble Fluid« von Eli Lilly und Col, Indianapolis, oder je 5 g natürliches Zitronenaroma und 5 g »Halb und »Halb-Essenz, beide von der Firma Haarmann und Reimer, Holzminden, Deutschland, zu, filtriert die erhaltene Lösung und ergänzt das Filtrat mit dest. Wasser auf 10 Liter.

d) Um 1000 Kapseln mit je 100 mg Wirkstoffgehalt herzustellen, mischt man 100 g Wirkstoff mit 173,0 g Lactose, befeuchtet die Mischung gleichmäßig mit einer wäßrigen Lösung von 2,0 g Gelatine und granuliert sie durch ein geeignetes Sieb (z. B. Sieb III nach Ph. Helv. V). Das Granulat mischt man mit 10,0 g getrockneter Maisstärke und 15,0 g Talk und füllt es gleichmäßig in 1000 Hartgelatinekapseln der Größe 1.

e) Als rektal anwendbare pharmazeutische Präparate kommen z. B. Suppositorien in Betracht, welche aus einer Kombination einer Verbindung der allgemeinen Formel I als Wirkstoff mit einer Suppositoriengrundmasse bestehen. Als Suppositoriengrundmasse eignen sich z. B. natürliche oder synthetische Triglyceride, Paraffinkohlenwasserstoffe, Polyäthylenglykole oder höhere Alkanole. Ferner können auch Gelatine-Rektalkapseln verwendet werden, die aus einer Kombination des Wirkstoffes mit einer Grundmasse bestehen; als Grundstoffe kommen z. B. flüssige Triglyceride, Polyäthylenglykole oder Paraffinkohlenwasserstoffe in Frage.

f) Zur parenteralen Verabreichung eignen sich in erster Linie Injektionslösungen der oben beschriebenen Salze. Injektionslösungen eines Hydrochlorids werden beispielsweise wie folgt hergestellt: 20,0 g Hydrochlorid eines Wirkstoffes werden in 1500 ml ausgekochtem, pyrogenfreiem Wasser gelöst und die Lösung mit ebensolchem Wasser auf 2000 ml ergänzt. Die Lösung wird abfiltriert, in 1000 Ampullen à 2 ml abgefüllt und sterilisiert. Eine Ampulle à 2 ml enthält 20 mg bzw. 1,0% Wirkstoff.

Die nachfolgenden Beispiele erläutern die Herstellung der neuen Verbindungen der allgemeinen Formel I und von bisher nicht beschriebenen Zwischenprodukten näher, stellen jedoch keineswegs die einzigen Ausführungsformen derselben dar. Die Temperaturen sind in Celsiusgraden angegeben.

## Beispiel 1

a) 54,3 g 1-Aminoäthyl-2-(2,6-dichloranilinomethyl)-2-imidazolin werden in 300 ml absolutem Toluol gelöst und bei Raumtemperatur mit 25,2 g p-Tolylisocyanat in 100 ml Toluol tropfenweise versetzt, wobei sich das Gemisch auf etwa 33° erwärmt. Anschließend rührt man 3 Stunden lang bei einer Temperatur von 90°, saugt von einer kleinen Menge einer kristallinen Trübung ab und engt das Reaktionsgemisch auf die Hälfte seines Volumens ein. Nach dem Anreiben kristallisiert der 1-[2-[2-(2,6-Dichloranilinomethyl)-2-imidazolin-1-yl]-äthyl]-3-(p-tolyl)-harnstoff mit einem Schmelzpunkt von 94 – 97° aus.
Das Maleinat wird erhalten, indem man 57,0 g des oben erhaltenen Harnstoffes in 250 ml Aceton löst und mit einer Lösung aus 15,8 g Maleinsäure in 100 ml Aceton versetzt. Nach kürzerem Stehenlassen kristallisiert das 1-[2-[2-(2,6-Dichloranilinomethyl)-2-imidazolin-1-yl]-äthyl]-3-(p-tolyl)-harnstoffmaleinat aus, welches nach dem Absaugen und Auswaschen bei einer Temperatur von 185 – 187° unter Zersetzung schmilzt.

b) Das für die Herstellung benötigte Ausgangsmaterial 1-Aminoäthyl-2-(2,6-dichloranilinomethyl)-2-imidazolin wird wie folgt hergestellt:
60,4 g 2,6-Dichloranilinoacetonitril und 34,0 g Diäthylentriamin werden in Gegenwart von 250 mg Schwefelwasserstoffgas erhitzt bis die Ammoniakentwicklung beendet ist. Die erhaltene freie Base, das 1-Aminoäthyl-2-(2,6-dichloranilinomethyl)-2-imidazolin wird zum Reinigen in Essigester gelöst und mit 2,5-n alkoholischer Salzsäure versetzt und so in das Dihydrochlorid mit einem Schmelzpunkt von 254 – 258° überführt. Aus dem Dihydrochlorid wird durch Lösen in Wasser und Fällen mit 10-n Natronlauge und anschließendem Ausäthern die gereinigte Base hergestellt.

## Beispiel 2

a) 22,4 g 1-Aminoäthyl-2-(2-chlorphenyl)-2-imidazolin, gelöst in 125 ml abs. Toluol, werden unter Eiswasserkühlung mit 13,3 g p-Tolylisocyanat in 50 ml abs. Toluol tropfenweise versetzt. Das durch die exotherm verlaufende Reaktion erwärmte Reaktionsgemisch wird 3 Stunden lang bei einer Temperatur von 90° gerührt, wobei sich eine ölige Substanz ausscheidet. Das als Lösungsmittel verwendete Toluol wird eingedampft, der Rückstand mit dem ausgeschiedenen Öl vereinigt, welches nach einigem Stehenlassen kristallisiert. Das erhaltene kristalline Produkt wird mit 200 ml warmem Essigester suspendiert und kurze Zeit zum Sieden erwärmt. Der hierbei als

unlöslich isolierte 1-[2-[2-(2-Chlorphenyl)-2-imidazolin-1-yl]-3-(p-tolyl)-harnstoff schmilzt bei 166—167°. Das Hydrochlorid wird erhalten, indem man 57,0 g des oben erhaltenen Harnstoffes in 100 ml Essigester suspendiert, mit 77,5 ml 2,08-n alkoholischer Salzsäure versetzt, die erhaltene Lösung filtriert und mit weiteren 100 ml Essigester versetzt. Das kristallin ausgeschiedene 1-[2-[2-(2-Chlorphenyl)-2-imidazolin-1-yl]-äthyl]-3-(p-tolyl)-harnstoff-hydrochlorid schmilzt bei 108—111° (ca. 1 Mol Kristallwasser enthaltend).

b) Das für die Umsetzung benötigte Ausgangsmaterial 1-Aminoäthyl-2-(2-chlorphenyl)-2-imidazolin wird in analoger Weise wie unter 1 b) beschrieben unter Verwendung von 68,8 g 2-Chlorbenzonitril, 57,9 g Diäthylentriamin und 300 mg Schwefelwasserstoffgas hergestellt; Siedepunkt 154°/0,02 Torr.

## Beispiel 3

a) In analoger Weise wie unter 2 a) beschrieben, werden aus 21,8 g 1-Aminoäthyl-2-(anilinomethyl)-2-imidazolin und 13,3 g p-Tolylisocyanat der 1-[2-[2-Anilinomethyl)-2-imidazolin-1-yl]-äthyl]-3-(p-tolyl)-harnstoff hergestellt, der nach dem Umkristallisieren aus Essigester und Äthanol bei einer Temperatur von 158—161° schmilzt. Das nach dem unter 2 a) beschriebenen Verfahren hergestellte Maleinat schmilzt bei einer Temperatur von 104—106°. Je nach Feuchtigkeitsgehalt steigt die Temperatur bis auf 115—118° an.

b) Das nach dem unter 1 b) beschriebenen Verfahren aus Anilinoacetonitril und Diäthylentriamin erhaltene 1-Aminoäthyl-2-(anilinomethyl)-2-imidazolin schmilzt nach Überführung in das Dihydrochlorid ungereinigt unter Zersetzung bei einer Temperatur von 106°. Zur Umsetzung wird daraus die freie Base durch Lösen mit Wasser und Fällen mit 10-n Natronlauge hergestellt.

## Beispiel 4

In analoger Weise wie unter 2 a) beschrieben, werden aus 21,1 g 1-Aminoäthyl-2-(2,6-dichloranilinomethyl)-2-imidazolin und 8,8 g Phenylisocyanat der 1-[2-[2-(2,6-Dichloranilinomethyl)-2-imidazolin-1-yl]-äthyl]-3-(phenyl)-harnstoff, der als Öl anfällt nach Überführung in das Maleinat in einem Gemisch aus Aceton und Essigester kristallin erhalten wird, Schmelzpunkt 163—165° unter Zersetzung.

## Beispiel 5

a) In analoger Weise wie unter 2 a) beschrieben, werden aus 25,2 g 1-Aminoäthyl-2-(2-chloranilinomethyl)-2-imidazolin und 18,7 g m-Trifluormethylphenylisocyanat der 1-[2-[2-(2-Chloranilinomethyl)-2-imidazolin-1-yl]-äthyl]-3-(3-trifluormethylphenyl)-harnstoff erhalten, der nach Suspension in Essigester bei einer Temperatur von 181—183° schmilzt. Das daraus hergestellte Hydrochlorid schmilzt bei einer Temperatur von 107°, dessen Kristallwasser beim Trocknen im Hochvakuum entfernt wird.

b) Das nach dem unter 1 b) beschriebenen Verfahren aus o-Chloranilinoacetonitril und Diäthylentriamin erhaltene 1-Aminoäthyl-2-(2-chloranilinomethyl)-2-imidazolin wird über das entsprechende Oxalat gereinigt.

## Beispiel 6

a) In analoger Weise wie unter 2 a) beschrieben, wird aus 25,3 g 1-Aminoäthyl-2-(4-chloranilinomethyl)-2-imidazolin und 18,7 g p-Trifluormethylphenylisocyanat der 1-[2-[2-(4-Chloranilinomethyl)-2-imidazolin-1-yl]-äthyl]-3-(4-trifluormethylphenyl)-harnstoff vom Schmelzpunkt 173—177° erhalten, der nach der Überführung in das Hydrochlorid bei einer Temperatur von 170—172° schmilzt.

b) Das nach dem unter 1 b) beschriebenen Verfahren aus p-Chloranilinoacetonitril und Diäthylentriamin erhaltene 1-Aminoäthyl-2-(4-chloranilinomethyl)-2-imidazolin schmilzt als Dihydrochlorid bei einer Temperatur von 204°. Daraus wird die Base als Öl erhalten.

## Beispiel 7

In analoger Weise wie unter 1 a) beschrieben, wird aus 25,3 g 1-Aminoäthyl-2-(2-chloranilinomethyl)-2-imidazolin und 13,3 g p-Tolylisocyanat der 1-[2-[2-(2-Chloranilinomethyl)-2-imidazolin-1-yl]-äthyl]-3-3-(p-tolyl)-harnstoff mit einem Schmelzpunkt von 142—144° erhalten. Das nach dem unter 1 a) beschriebenen Verfahren hergestellte Maleinat schmilzt bei einer Temperatur von 126—127°.

## Beispiel 8

In analoger Weise wie unter 1 a) beschrieben wird aus 21,8 g 1-Aminoäthyl-2-(anilinomethyl)-2-imidazolin und 15,4 g m-Chlorphenylisocyanat der 1-[2-[2-(Anilinomethyl)-2-imidazolin-1-yl]-äthyl]-3-(3-chlorphenyl)-harnstoff mit einem Schmelzpunkt von 140—143° erhalten. Das nach dem unter 2 beschriebenen Verfahren hergestellte Maleinat schmilzt bei einer Temperatur von 111—113°.

## Beispiel 9

In analoger Weise wie unter 2 a) beschrieben, wird aus 21,4 g 1-Aminomethyl-2-(anilinomethyl)-2-imidazolin und 18,7 g m-Trifluormethylphenylisocyanat der 1-[2-[2-1Anilinomethyl)-2-imidazolin-1-yl]-äthyl]-3-(3-trifluormethylphenyl)-harnstoff mit einem Schmelzpunkt von 128—130° erhalten.

## Beispiel 10

In analoger Weise wie unter 2 a) beschrieben, werden aus 22,4 g 1-Aminoäthyl-2-(2-chlorphenyl)-2-imidazolin und 15,4 g 4-Chlorphenylisocyanat der 1-[2-[2-(2-Chloranilinomethyl)-2-imidazolin-1-yl]-äthyl]-3-(4-chlorphenyl)-harnstoff mit einem Schmelzpunkt von 77—80° erhalten.
Das daraus hergestellte Hydrochlorid schmilzt bei einer Temperatur von 121°.

## Beispiel 11

Wie unter Beispiel 1 a) beschrieben, werden in analoger Weise aus 25,3 g 1-Aminoäthyl-2-(4-chloranilinomethyl)-2-imidazolin und 13,3 g p-Tolylisocyanat der 1-[2-[2-(4-Chloranilinomethyl)-2-imidazolin-1-yl]-äthyl]-3-(4-tolyl)-harnstoff mit einem Schmelzpunkt von 176—179° erhalten.
Das daraus hergestellte Hydrochlorid schmilzt bei einer Temperatur von 113°.

## Beispiel 12

a) In analoger Weise wie unter 1 a) beschrieben, werden aus 26,1 g 1-Aminoäthyl-2-(2,6-dimethylanilinomethyl)-2-imidazolin und 15,6 g p-Tolylisocyanat der 1-[2-[2-(2,6-Dimethylanilinomethyl)-2-imidazolin-1-yl]-äthyl]-3-(4-tolyl)-harnstoff erhalten, dessen Reinigung über das Dihydrochlorid in folgender Weise stattfindet: Die als Rückstand erhaltene freie Base wird in Essigester gelöst und mit zwei Äquivalenten 2,76-n alkoholischer Salzsäure versetzt. Mit Äther wird das Dihydrochlorid gefällt, welches als Öl anfällt. Nach dem Abgiessen des Lösungsmittels wird der Rückstand in ca. 50 ml Isopropanol gelöst und mit ca. 1000 ml Äther nochmals gefällt. Nach nochmaliger Widerholung dieser Prozedur wird das Dihydrochlorid nur als semikristallines Produkt erhalten, welches keinen definitiven Schmelzpunkt (Schaumbildung) aufweist.

b) Das für die Umsetzung benötigte Ausgangsmaterial 1-Aminoäthyl-2-(2,6-dimethylanilinomethyl)-2-imidazolin wird wie folgt hergestellt:
48,0 g 2,6-Dimethylanilinoacetonitril und 34,6 g Diäthylentriamin werden in Gegenwart von 300 mg Schwefelwasserstoffgas erhitzt bis die Ammoniakentwicklung beendet ist. Das erhaltene Reaktionsgemisch wird in Benzol aufgenommen und das Lösungsmittel und die Ausgangsmaterialien bis 80°/0.1 Torr abdestilliert und der Rückstand über das Dihydrochlorid gereinigt. Das erhaltene 1-Aminoäthyl-2-(2,6-dimethylanilinomethyl)-2-imidazolin-dihydrochlorid schmilzt bei einer Temperatur von 200—203°. Zur Umsetzung wird daraus in Wasser und starker Lauge die Base freigesetzt.

## Beispiel 13

a) In analoger Weise wie unter 2 a) beschrieben, werden aus 20,3 g 1-Aminoäthyl-2-benzyl-2-imidazolin und 13,3 g p-Tolylisocyanat der 1-[2-[2-Benzyl-imidazolin-1-yl]-äthyl]-3-(4-tolyl)-harnstoff mit einem Schmelzpunkt von 149—151° erhalten. Das Hydrochlorid schmilzt bei 155—157°.

b) Das für die Umsetzung benötigte Ausgangsmaterial 1-Aminoäthyl-2-benzyl-2-imidazolin wird in folgender Weise hergestellt:
58,6 g Benzylcyanid und 57,9 g Diäthylentriamin werden in Gegenwart von 300 mg Schwefelwasserstoffgas 6 Stunden auf 115° erhitzt. Das Reaktionsgemisch wird in 100 ml Benzol aufgenommen und portionenweise mit insgesamt 500 ml kalter 2-n Salzsäure ausgezogen. Die salzsaure Lösung wird anschließend in 500 ml 10-n Natronlauge eingegossen und das Lösungsgemisch mit Dichlormethan extrahiert. Der Dichlormethanextrakt wird im Vakuum destilliert, wobei das 1-Aminoäthyl-2-benzyl-2-imidazolin einen Siedepunkt von 135—142° bei 0,03 Torr aufweist.

## Beispiel 14

a) 32,5 g 1-Aminoäthyl-2-(2-hydroxyphenyl)-2-imidazolin werden in 200 ml Toluol gelöst und bei 20–26° mit 20,0 g p-Tolylisocyanat in 75 ml Toluol tropfenweise versetzt. Das Gemisch aus dem sich eine ölige Phase abtrennt, wird 3 Std. bei 90° gerührt, im Vakuum so weit wie möglich eingedampft, der Rückstand in 100 ml Aceton gelöst und mit 60 ml alkoholischer Salzsäure (2,08N) versetzt. Man gibt 100 ml abs. Äther zu und überläßt die Lösung der Kristallisation. Das isolierte und mit Isopropanol/Aceton gewaschene 1-[2-[2-(2-Hydroxyphenyl)-2-imidazolinyl]-äthyl]-3-(4-tolyl)-harnstoff-hydrochlorid schmilzt bei 115° (Zers.)

b) Das für die Umsetzung benötigte Ausgangsmaterial 1-Aminoäthyl-2-(2-hydroxyphenyl)-2-imidazolin wird wie folgt hergestellt:
66,5 g 2-Methoxybenzonitril und 56,75 g Diäthylentriamin werden mit 500 mg Schwefelkohlenstoff 8 Stunden im Bad bei einer Außentemperatur von 130° gerührt. Nach Beendigung der Gasentwicklung wird das Reaktionsgemisch in Toluol aufgenommen, dieses zusammen mit nicht umgesetzten Diäthylentriamin in Vakuum unter Rotation verdampft und der Rückstand im Hochvakuum destilliert; Siedepunkt: 145–150°/0,02 Torr oder 135°/0,01 Torr. Die Verbindung ist wasserlöslich und mit konzentrierter Natronlauge nicht fällbar. Die Methoxygruppe wurde während der Reaktion zur Hydroxygruppe aufgespalten und so das 1-Aminoäthyl-2-(2-hydroxyphenyl)-2-imidazolin erhalten.

## Beispiel 15

a) 21,9 g 1-Aminoäthyl-2-(4-methoxyphenyl)-2-imidazolin werden in 100 ml Toluol vorgelegt und, wie im Beispiel 2 beschrieben, mit 13,3 g p-Tolylisocyanat in 75 ml Toluol der 1-[2-[2-(4-Methoxyphenyl)-2-imidazolinyl)-äthyl]-3-(4-tolyl)-harnstoff vom Schmelzpunkt 66–70° (aus Essigester-Äther) erhalten. Er enthält Kristallwasser und wird aus Essigester nochmals umkristallisiert, wobei der Schmelzpunkt der wasserfreien Base erhalten wird: 134–136°.
Die Salze sind meist hygroskopisch bzw. enthalten Kristallwasser und weisen einen sehr tiefen Schmelzpunkt auf. Aus konzentrierter, wäßriger Lösung, die 1 Äquivalent Salzsäure enthält, kristallisiert das Hydrochlorid in Plättchen.

b) Das für die Umsetzung benötigte Ausgangsmaterial 1-Aminoäthyl-2-(4-methoxyphenyl)-2-imidazolin wird analog den Beispielen 2 bzw. 14 aus 39,9 g 4-Methoxybenzonitril und 34,0 g Diäthylentriamin und 0,5 ml Schwefelkohlenstoff bei 110° erhalten. Eine nachfolgende Destillation liefert 1-Aminoäthyl-2-(4-methoxyphenyl)-2-imidazolin mit einem Siedepunkt von 144–147°/0,04 Torr. Die p-Methoxygruppe wird nicht zur p-Hydroxygruppe aufgespalten.

## Beispiel 16

a) 19,0 g 1-Aminoäthyl-2-(4-pyridyl)-2-imidazolin und 13,3 g p-Tolylisocyanat werden, wie in den Beispielen 2 und 15, umgesetzt. Durch Zugabe von 200 ml Essigester nach beendeter Reaktion wird der 1-[2-[-2-(4-Pyridyl)-2-imidazolinyl]-äthyl]-3-(4-tolyl)-harnstoff in kristalliner Form abgeschieden. Nach dem Abtrennen wird das erhaltene Produkt aus Essigester umkristallisiert, Smp. 109–111°.

b) Das für die Umsetzung benötigte Ausgangsmaterial 1-Aminoäthyl-2-(4-pyridyl)-2-imidazolin wird analog den Beispielen 2 und 15 aus 52 g 4-Cyan-pyridin und 56,75 g Diäthylentriamin in Gegenwart von 1 ml Schwefelkohlenstoff als Katalysator in leicht exothermer Reaktion, Ölbad 110°, Sdp. 130–132°/0,015 Torr, erhalten.

## Beispiel 17

a) 19,0 g 1-Aminoäthyl-2-(2-pyridyl)-2-imidazolin und 13,3 g p-Tolylisocyanat werden in Toluol als Lösungsmittel wie in den Beispielen 2 und 16 kondensiert. Das während der Reaktion kristallisierende Produkt wird nach 3 Stunden abgesaugt und aus 400 ml Essigester umkristallisiert. Der erhaltene 1-[2-[2-(2-Pyridyl)-2-imidazolinyl]-äthyl]-3-(4-tolyl)-harnstoff schmilzt bei 165–167°.
Aus alkoholischer Lösung wird mit einem Äquivalent alkoholischer 2,6-n Salzsäure durch Zugabe von Essigester ein kristallines Monohydrochlorid erhalten, smp. 193–195°.

b) Das benötigte Ausgangsmaterial 1-Aminoäthyl-2-(2-pyridyl)-2-imidazolin wird analog Beispiel 16 aus 52 g 2-Cyanpyridin, 56,75 g Diäthylentriamin und 1 ml Schwefelkohlenstoff unter Rühren bei 110° (Badtemperatur) und direkte Destillation des Rohproduktes, Sdp. 124–127/0,04 Torr erhalten.

## Beispiel 18

22,4 g 1-Aminoäthyl-2-(2-chlorphenyl)-2-imidazolin (erhalten nach Beispiel 2 b) werden in 100 ml Toluol vorgelegt und bei 20–30° 15,4 g p-Methylbenzoylchlorid in 100 ml Toluol langsam zugetropft. Es entsteht hierbei eine Suspension, die 3 Stunden bei 90° (Badtemperatur) gehalten und dann gekühlt wird. Nach 12 Stunden ist das abgeschiedene Öl durchkristallisiert. Der kristalline Anteil wurde folgendermaßen gereinigt: Man löst ihn in 200 ml Wasser, filtriert die trübe Lösung über Celite, fällt die Base mit gesättigter Kaliumcarbonatlösung aus und nimmt sie in Methylenchlorid auf. Letzteres liefert nach Trocknen und Verdampfen im Vacuum ein zähes Öl, das in 50 ml Essigester gelöst und mit 32,5 ml 2,65-n alkoholisches HCl versetzt wird. Die Lösung wird weitgehend eingedampft und der Rückstand in 200 ml Aceton gelöst, wobei Kristallisation eintritt. Man saugt den Niederschlag ab und wäscht mit Aceton und erhält so das 1-[2-(4-Methyl-benzoylamino)-äthyl]-2(2-chlorphenyl)-2-imidazolin-hydrochlorid vom Smp. 100° (Zers.). Es enthält Kristallwasser und schmilzt nach längerem Trocknen im Hochvacuum bei 120°.

## Beispiel 19

67,2 g 1-Aminoäthyl-2-(2-chlorphenyl)-2-imidazolin (erhalten nach Beispiel 2 b) werden in 400 ml Toluol gelöst und bei Raumtemperatur mit 57,3 g p-Isocyanato-benzoesäure-äthylester in 150 ml Toluol tropfenweise versetzt. Die Reaktion wird durch 3stündiges Rühren bei 90° (Badtemperatur) vervollständigt. Die Toluollösung wird vom abgeschiedenen Öl dekantiert und mit Toluol gewaschen, dann in Methylenchlorid aufgenommen, mit Wasser gewaschen, und am Rotationsverdampfer eingedampft. Der Rückstand wird in 200 ml Essigester gelöst und mit 126 ml 2,37-n alkoholischer Salzsäure versetzt. Bei der Zugabe von 500 ml Essigester kristallisiert das 1-[2-[2-(2-Chlorphenyl)-2-imidazolin-1-yl]-äthyl]-3-(4-äthoxycarbonylphenyl)-harnstoff-hydrochlorid, das nach dem Umkristallisieren aus Isopropanol-Aceton ab 110° unter langsamer Zersetzung und Aufschäumen bei 140° schmilzt.

## Beispiel 20

22,6 g des nach Beispiel 19 erhaltenen 1-[2-[2-(Chlorphenyl)-2-imidazolin-1-yl]-äthyl]-3-(4-äthoxycarbonyl-phenyl)-harnstoff-hydrochlorid wird in 900 ml 95%igem Alkohol gelöst und mit 50 ml 1-n Natronlauge die Base freigesetzt. Nach Zugabe von 200 ml 2-n Natronlauge wird über Nacht gerührt. Es wird nun mit 200 ml 2-n Salzsäure neutralisiert und mit 51 ml 1-n Salzsäure schwach sauergestellt. Man verdampft unter Rotation zur Trockne und kocht den Rückstand zweimal mit 100 ml Alkohol aus. Der Rückstand besteht aus Natriumchlorid; die Lösung liefert nach weitgehendem Einengen das kristalline 1-[2-[2-(2-Chlorphenyl)-2-imidazolin-1-yl]-äthyl]-3-(4-carboxyphenyl)-harnstoff-hydrochlorid, das aus Methanol-Isopropanol umkristallisiert wird und dann bei 240–242° unter Zersetzung schmilzt.

## Beispiel 21

16,8 g 1-Aminoäthyl-2-(2-pyridyl)-2-imidazolin (hergestellt nach Beispiel 17 b) in 100 ml Toluol und 16,9 g 4-Isocyanato-benzoesäureäthylester in 50 ml Toluol werden wie im Beispiel 19 kondensiert. Der kristallin anfallende 1-[2-[2-(2-Pyridyl)-2-imidazolin-1-yl]-äthyl]-3-(4-äthoxycarbonyl-phenyl)-harnstoff schmilzt bei 152–154°. Es wird nach Beispiel 1 a) in das Maleinat vom Schmelzpunkt 95–97° Zers. übergeführt.

## Beispiel 22

373 g 1-Aminoäthyl-2-(4-pyridyl)-2-imidazolin (hergestellt in Analogie zu Beispiel 17 b), Siedepunkt 130–132°/0,015 Torr werden in 2 l Toluol gelöst und bei 20–26° unter starkem Rühren mit 375 g 4-Isocyanatobenzoesäureäthylester in 1 l Toluol zugetropft. Es entsteht eine ölige Fällung, die bei weiterer 3stündiger Reaktionsdauer bei einer Temperatur von 90° kristallin wird. Die Suspension wird abgesaugt, mit Toluol und Äther gewaschen und getrocknet. Man erhält 681 g 1-[2-[2-(4-Pyridyl)-2-imidazolin-1-yl]-äthyl]-3-(4-äthoxycarbonyl-phenyl)-harnstoff vom Schmelzpunkt 120°. Das Dihydrochlorid wird aus Aceton durch Umsetzung mit 2 Äquivalenten alkoholischer Salzsäure erhalten.

Beispiel 23

a) 68,1 g 1-[2-[2-(4-Pyridyl)-2-imidazolin-1-yl]-äthyl-3-(4-äthoxycarbonyl-phenyl)-harnstoff werden in 1,3 l 95% Äthanol gelöst, mit 0,3 l Natronlauge (28,1 g feste Natronlauge enthaltend) versetzt und anschließend 15 Stunden lang bei Raumtemperatur gerührt. Die alkalische Lösung wird mit der äquivalenten Menge (42,0 g) Eisessig filtriert und die klare Lösung wird im Vakuum auf ca. 1/3 des Volumens eingeengt. Der hierbei erhaltene kristalline Niederschlag wird isoliert und mit Wasser und Isopropanol gewaschen. Der erhaltene 1-[2-[2-(4-Pyridyl)-2-imidazolin-1-yl]-äthyl]-3-(4-carboxy-phenyl)-harnstoff schmilzt bei 181—184° unter Zersetzung.

b) Das Hydrochlorid wird wie folgt hergestellt:
42,9 g der oben erhaltenen Base werden in einem Gemisch aus 100 ml Methanol und 100 ml Wasser suspendiert und mit 1 Äquivalent 2,4-n alkoholischer Salzsäure versetzt. Die erhaltene Lösung wird filtriert und auf ca. 1/3 des Volumens im Vakuum eingeengt, wobei ein kristalliner Niederschlag anfällt. Durch Zugabe von 100 ml Isopropanol wird die Kristallisation vervollständigt. Das erhaltene Hydrochlorid schmilzt bei 217° unter Zersetzung.

Beispiel 24

In gleicher Weise wie im Beispiel 23 beschrieben, wird der nach Beispiel 21 erhaltene 1-[2-[2-(2-Pyridyl-2-imidazolin-1-yl]-äthyl]-3-(4-äthoxycarbonylphenyl)-harnstoff verseift, wobei der 1-[2-[2-(Pyridyl)-2-imidazolin-1-yl]-äthyl]-3-(4-carboxy-phenyl)-harnstoff anfällt. Es schmilzt bei 220—223°. Das Hydrochlorid schmilzt bei 238—240° (unter Zersetzung).

Beispiel 25

22,4 g 1-Aminoäthyl-2-(2-chlorphenyl)-2-imidazolin werden in 150 ml Toluol und 13,5 g Phenylisothiocyanat in 75 ml Toluol zugetropft. Es fällt dabei ein öliger Niederschlag aus. Man rührt 3 Stunden bei 90°, kühlt, trennt das Toluol vom Niederschlag ab, löst das zurückbleibende Öl in Methylenchlorid und wäscht dieses in Wasser. Aus dem Methylenchlorid gewinnt man als Rückstand ein Öl, welches nach chromatographischer Reinigung über Kieselgel (450 g, Fließmittel: Aceton) den 1-[2-[2-(2-Chlorphenyl)-2-imidazolin-1-yl]-äthyl]-3-phenylthioharnstoff ergibt. Aus der Base wird in alkoholischer Lösung mit alkoholischer Oxalsäure das saure Oxalat in kristalliner Form erhalten, welches unter Zersetzung bei 142° schmilzt.

Aus dem erhaltenen Oxalat kann durch Umsetzung mit 2-n Natronlauge die Base in kristalliner Form erhalten werden, die in Acetonlösung durch Umsetzung mit der berechneten Menge alkoholischer 2,5-n Salzsäure in das Hydrochlorid umgewandelt wird. Durch Einengen der Reaktionslösung und Zugabe von Tetrahydrofuran fällt das Hydrochlorid kristallin an und schmilzt mit 0,5 Mol Kristallwasser bei 93°.

Beispiel 26

a) 29,1 g 1-(3-Aminopropyl)-2-(4-pyridyl)-1,4,5,6-tetrahydropyrimidin werden in 150 ml Toluol gelöst und mit 25,5 g 4-Isocyanato-benzoesäureäthylester in 100 ml Toluol langsam versetzt. Es entsteht eine ölige Fällung. Das Gemisch wird 3 Stunden bei 90° gerührt, das Toluol abgegossen und das erhaltene Öl in Methylenchlorid gelöst, filtriert und das Lösungsmittel im Vakuum entfernt. Das quantitativ erhaltene, sehr langsam kristallisierende Öl wird direkt verseift. Die Verseifung wird in alkoholischer Lösung mit Natronlauge bei Raumtemperatur ausgeführt. Die alkalische Lösung wird mit Eisessig neutralisiert und anschließend im Vakuum weitgehend eingeengt. Beim Kühlen kristallisiert der 1-[3-[2-(4-(Pyridyl)-1,4,5,6-tetrahydropyrimidin-1-yl]-propyl]-3-(4-carboxyphenyl)-harnstoff aus, der bei 262° unter Zersetzung schmilzt. Das Hydrochlorid wird beispielsweise erhalten, indem man 16,2 g der Base in heißem Isopropanol mit 17,9 ml 2,26-n alkoholischer Salzsäure versetzt. Das anfallende Kristallisat schließt Lösungsmittel ein und schmilzt bei 123° unter Zersetzung. Die lösungsmittelfreie Substanz erhält man durch Erhitzen im Hochvakuum.

b) Das als Ausgangsmaterial verwendete 1-(3-Aminopropyl)-2-(4-pyridyl)-1,4,5,6-tetrahydropyrimidin wird analog zu Beispiel 16 b) aus 26 g 4-Cyanpyridin, 36 g Dipropylentriamin und 0,5 ml Schwefelkohlenstoff als Katalysator durch Erhitzen im Ölbad auf 110° erhalten, welches bei einer Temperatur von 154—155°/0,015 Torr siedet.

Beispiel 27

22,0 g 2-Chlor-benzimidoäthyläther-hydrochlorid (hergestellt aus 2-Chlorbenzonitril und einem Äquivalent Äthylalkohol in Chloroform und Sättigen der erhaltenen Lösung mit Salzsäuregas) und 23,6 g 1-[1-(1-Aminoäthyl-(2)-amino)-äthyl(2)]-3-(p-tolyl)-harnstoff (hergestellt durch langsames Zutropfen von 1 Mol p-Tolylisocyanat in eine Lösung von 3 Mol Diäthylentriamin in Toluol werden in 150 ml Äthylalkohol zur Beendigung der Ammoniakentwicklung unter Rückfluß zum Sieden erhitzt. Die alkoholische Lösung wird auf ein geringes Volumen im Vakuum eingedampft und portionenweise mit einem Gemisch von Aceton-Essigester (1 : 1) versetzt und ausgerieben, wobei der im Beispiel 2 beschriebene 1-[2-[2-(2-Chlorphenyl)-2-imidazolin-1-yl]-äthyl]-3-(p-tolyl)-harnstoff als Hydrochlorid kristallin anfällt, Smp. 108°. Die durch Kaliumcarbonat freigesetzte Base schmilzt bei einem Schmelzpunkt von 166–168.

Beispiel 28

a) 34,4 g 1-Aminoäthyl-2-[(2,6-dimethyl-4-pyrimidyl)-amino]-2-imidazolin werden in 150 ml Essigester vorgelegt und bei 20–24° 27,7 g 4-Isocyanato-benzosäureäthylester in 100 ml Essigester zugetropft. Man rührt die allmählich kristallisierende Lösung 3 Std. bei einer Badtemperatur von 80°, saugt die erhaltenen Kristalle ab und wäscht mit Essigester, wobei der 1-[2-[2-(N-2,6-Dimethyl-4-pyrimidinyl)-N-(4-äthoxycarbonylphenylcarbamoylamino)-2-imidazolin-1-yl)-3-äthyl]-(4-äthoxycarbonyl-phenyl-harnstoff anfällt, der einen Schmelzpunkt von 168–171° aufweist.
Die verbliebene Mutterlauge wird im Vakuum eingedampft, der Rückstand mit 300 ml 1-n Salzsäure angerührt und die Salzsäure mit dem gleichen Volumen Wasser verdünnt. Vom ungelösten Teil wird abgegossen und die trübe Lösung durch Filtration über Aktivkohle geklärt. Das Filtrat wird mit 150 ml gesättigter Kaliumkarbonatlösung versetzt und die alkalische Lösung mehrfach mit Chloroform extrahiert. Der Chloroformextrakt liefert nach üblicher Aufbearbeitung den 1-[2-[2-(2,6-Dimethyl-4-pyrimidyl-amino)-2-imidazolidin-1-yl]-äthyl]-3-(4-äthoxycarbonyl-phenyl)-harnstoff als zähes Harz. Dieser wird in 200 ml Aceton gelöst und mit 49 ml äthanolischer 2,4 N Salzsäure neutralisiert. Nach Zugabe von etwa 100 ml Essigester beginnt die kristallisation. Das so erhaltene 1-[2-[2-(2,6-Dimethyl-4-pyrimidyl-amino)-2-imidazolin-1-yl]-äthyl]-3-(4-äthoxycarbonyl-phenyl)-harnstoff-hydrochlorid wird isoliert und mit Essigester gewaschen. Es hat einen Schmelzpunkt von 218–220°.
Das als Ausgangsmaterial verwendete 1-Aminoäthyl-2-[(2,6-dimethyl-4-pyrimidyl)-amino]-2-imidazolin wird folgendermaßen erhalten:

b) 24,6 g 4-Amino-2,6-dimethylpyrimidin werden in 100 ml Chloroform mit 26,3 g Äthoxycarbonylisothiocyanat 40 Minuten unter Rückfluß gerührt. Die erhaltenen Kristalle des 3-Äthoxycarbonyl-1-(2,6-dimethyl-4-pyrimidyl)-thioharnstoffs schmelzen nach Kristallisation aus 90%igem Alkohol bei 162–165°.

c) 36 g des nach b) erhaltenen Äthoxycarbonylthioharnstoffs werden in 200 ml 1-n Natronlauge 1 Std. unter Rückfluß und Rühren gekocht. Die abgeschiedenen Kristalle des 1-(2,6-Dimethyl-4-pyrimidinyl)-thioharnstoffs schmelzen bei 236–238°.

d) 20,4 g des nach c) erhaltenen Thioharnstoffs werden mit 16,7 g Methyljodid in 130 ml Methanol 1 Std. unter Rückfluß und Rühren gekocht. Man erhält quantitativ das 1-(2,6-Dimethyl-4-pyrimidyl)-S-methylisothiuronium-jodid vom Smp. 196–200°.

e) 96 g des nach d) erhaltenen Isothiuroniumsalzes werden mit 61 g Diäthyltriamin in 600 ml Methanol 6 Std. unter Rühren und Rückfluß gekocht. Es wird Methylmercaptan und Ammoniak abgespalten. Die Lösung wird zur Trockene eingeengt, der Rückstand in 300 ml Wasser gelöst, die trübe Lösung klar filtriert und erschöpfend (d. h. 7mal) mit Chloroform extrahiert. Der Extrakt liefert das 1-Aminoäthyl-2-[(2,6-dimethyl-4-pyrimidinyl)-amino]-2-imidazolin als gelbes Öl, das über sein Dihydrochlorid vom Smp. 208–214° gereinigt wird.

Beispiel 29

In gleicher Weise wie in Beispiel 23 beschrieben, wird der nach Beispiel 28 erhaltene 1-[2-[2-(2,6-Dimethyl-4-pyrimidinyl-amino)-2-imidazolin-1-yl]-äthyl]-3-(4-äthoxycarbonyl-phenyl)-harnstoff verseift und so der 1-[2-[2-(2,6-Dimethyl-4-pyrimidinylamino)-2-imidazolin-1-yl]-äthyl]-3-(4-carboxy-phenyl)-harnstoff erhalten.
Smp. 160°. Bei 180° erfolgt Zersetzung. Das Hydrochlorid schmilzt bei 239–240° (Zersetzung).

Beispiel 30

In gleicher Weise wie in Beispiel 28 beschrieben, wird 1-Aminoäthyl-2-(2-pyridyl-amino)-2-imidazolin in Essigester mit 4-Isocyanato-benzoesäureäthylester umgesetzt und liefert den kristallinen 1-[2-[2-(2-Pyridylamino)-2-imidazolin-1-yl]-äthyl]-3-(4-äthoxycarbonyl-phenyl)-harnstoff.

Beispiel 31

In analoger Weise wie in Beispiel 22 beschrieben, erhält man aus 3-(Cyanmethyl)-pyridin das 1-Aminoäthyl-2-(3-pyridylmethyl)-2-imidazolin- und daraus mit 4-Isocyanato-benzoesäureäthylester den 1-[2-[2-(3-Pyridylmethyl)-2-imidazolin-1-yl]-äthyl]-3-(4-äthoxycarbonyl-phenyl)-harnstoff, Smp. 158,5°. Das Dihydrochlorid schmilzt bei 210° (Zersetzung).

Beispiel 32

In analoger Weise wie in den Beispielen 23 und 29 beschrieben, wird der nach Beispiel 31 erhaltene Äthylester verseift und so der 1-[2-[2-(3-Pyridylmethyl)-2-imidazolin-1-yl]-äthyl]-3-(4-carboxyphenyl)-harnstoff erhalten.

**Patentansprüche**

1. Neue Harnstoff- und Amidoverbindungen der allgemeinen Formel I

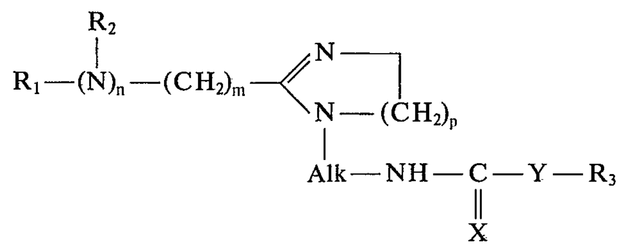

(I)

in der $R_1$ und $R_3$ ein monocyclisches, carbocyclisches Aryl oder Heteroaryl, n 0 oder 1, m 0, 1 oder 2, p 1 oder 2, $R_2$ ein Wasserstoffatom oder Niederalkyl, Alk eine Niederalkylengruppe mit 2—3 Kohlenstoffatomen in der Kettenlinie, X Sauerstoff oder Schwefel und Y die Iminogruppe oder eine direkte Bindung, bedeuten und deren Salze.

2. Neue Verbindungen der Formel I gemäß Anspruch 1, in der $R_1$, $R_2$, Alk, X, Y, n, m und p die im Anspruch 1 erwähnten Bedeutungen haben, mit der Maßgabe, daß, wenn m 0 bedeutet, n für 0 steht, und deren Salze.

3. Neue Verbindungen der Formel I gemäß Anspruch 1, in der $R_1$ und $R_3$ ein gegebenenfalls substituiertes Phenyl oder Pyridyl, X, Y, n, m und p die im Anspruch 1 erwähnten Bedeutungen haben, mit der Maßgabe, daß, wenn m 0 bedeutet, n für 0 steht, $R_2$ ein Wassserstoffatom, Alk eine Niederalkylengruppe mit 2 Kohlenstoffatomen in der Kettenlinie bedeuten, und deren Salze.

4. Neue Verbindungen der Formel I gemäß Anspruch 1, in der $R_1$ und $R_3$ ein gegebenenfalls durch Halogen, Niederalkyl, Carboxyl oder Trifluormethyl substituiertes Phenyl oder Pyridyl, n 0 oder 1, m 0 oder 1, p 1 bedeuten, mit der Maßgabe, daß, wenn m 0 bedeutet, n für 0 steht, $R_2$ ein Wasserstoffatom, Alk eine Niederalkylengruppe mit 2 Kohlenstoffatomen in der Kettenlinie, X Sauerstoff und Y die Iminogruppe oder eine direkte Bindung bedeuten und deren Salze.

5. Verbindungen der Formel I gemäß Anspruch 1, in der $R_1$ und $R_3$ ein gegebenenfalls durch Chlor, Methyl, Carboxyl oder Trifluormethyl substituiertes Phenyl oder Pyridyl-2- oder 4, n, m und p die im Anspruch 4 angegebenen Bedeutungen haben, $R_2$ ein Wasserstoffatom, Alk eine 1,2-Äthylengruppe, X Sauerstoff und Y die Iminogruppe bedeuten und deren therapeutisch verwendbare Salze.

6. 1-[2-[2-(2-Chlorphenyl)-2-imidazolin-1-yl]-äthyl]-3-(4-tolyl)-harnstoff.

7. 1-[2-[2-(4-Pyridyl)-2-imidazolin-1-yl]-äthyl]-3-(4-tolyl)-harnstoff.

8. 1-[2-[2-(2-Pyridyl)-2-imidazolin-1-yl]-äthyl]-3-(4-äthoxycarbonyl-phenyl)-harnstoff.

9. 1-[2-[2-(4-Pyridyl)-2-imidazolin-1-yl]-äthyl]-3-(4-äthoxycarbonyl-phenyl)-harnstoff.

10. 1-[2-[2-(4-Pyridyl)-2-imidazolin-1-yl]-äthyl]-3-(4-carboxyphenyl)-harnstoff.

11. 1-[2-[2-(2-Pyridyl)-2-imidazolin-1-yl]-äthyl]-3-(4-carboxyphenyl)-harnstoff.

12. Die in einem der Ansprüche 6 bis 11 genannten Verbindungen in Form ihrer therapeutisch verwendbaren Salze.

13. Pharmazeutische Präparate enthaltend eine der Verbindungen der Ansprüche 1—12.

14. Die Verbindungen der Ansprüche 1—12 zur Anwendung in einem Verfahren zur therapeutischen

Behandlung des menschlichen oder tierischen Körpers.

15. Die Verbindungen der Ansprüche 1—12 zur Verwendung als antitumor-wirksame Mittel.

16. Verwendung der Verbindungen der Ansprüche 1—12 zur Herstellung von pharmazeutischen Präparaten.

17. Verfahren zur Herstellung neuer Verbindungen der Formel I

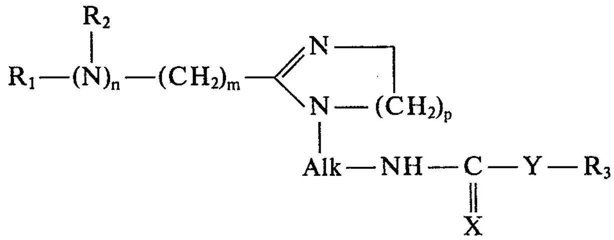

(I)

in der $R_1$ und $R_3$ ein monocyclisches, carbocyclisches Aryl oder Heteroaryl, n 0 oder 1, m 0, 1 oder 2, p 1 oder 2, $R_2$ ein Wasserstoffatom oder Niederalkyl, Alk eine Niederalkylengruppe mit 2—3 Kohlenstoffatomen in der Kettenlinie, X Sauerstoff oder Schwefel, Y die Iminogruppe oder eine direkte Bindung bedeuten und deren Salze, dadurch gekennzeichnet, daß man

a) eine Verbindung der allgemeinen Formel II

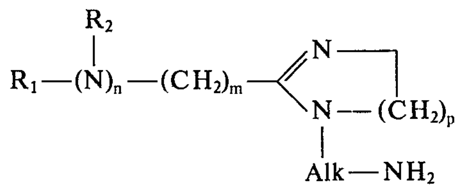

(II)

mit einem reaktionsfähigen Derivat der Säure der allgemeinen Formel III

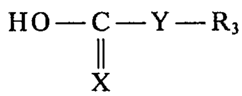

(III)

in der X, Y und $R_3$ die oben angegebenen Bedeutungen haben, umsetzt, oder

b) eine Verbindung der allgemeinen Formel VI

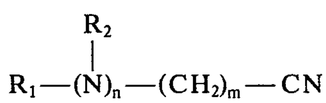

(VI)

in der $R_1$, $R_2$, n und m die oben angegebenen Bedeutungen haben, mit einem Diäthylen- bzw. Dipropylentriaminderivat der allgemeinen Formel VII

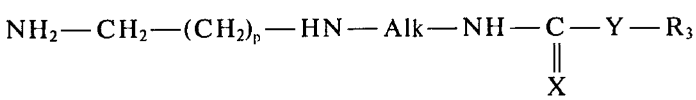

(VII)

in der p, X, Y, Alk und $R_3$ die oben angegebenen Bedeutungen haben, umsetzt, und eine erhaltene Verbindung der allgemeinen Formel I gewünschtenfalls im Rahmen der Definition der Endstoffe Substituenten einführt, abwandelt oder abspaltet, und/oder erhaltene Racematgemische in die reinen Racemate, und/oder erhaltene Racemate in die optischen Antipoden auftrennt, und/oder erhaltene Salze in die freien Verbindungen oder andere Salze oder erhaltene freie Verbindungen in ihre Salze umwandelt.

18. Die nach dem Verfahren des Anspruchs 17 erhältlichen Verbindungen.

**Claims**

1. A novel urea and amido compound of the general formula I

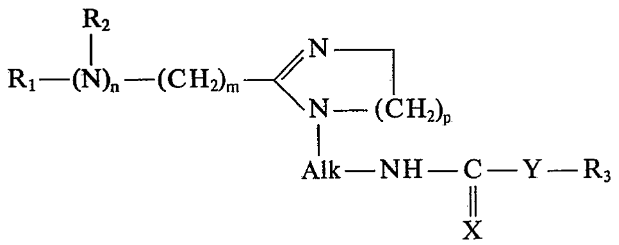

(I)

in which $R_1$ and $R_3$ are a monocyclic, carbocyclic aryl or hetero-aryl, n is 0 or 1, m is 0, 1 or 2, p is 1 or 2, $R_2$ is a hydrogen atom or lower alkyl, Alk is a lower alkylene group having 2—3 carbon atoms in the linear chain, X is oxygen or sulfur, and Y is an imino group or a direct bond, or a salt thereof.

2. A novel compound of the formula I according to Claim 1, in which $R_1$, $R_2$, Alk, X, Y, n, m and p have the meanings defined in Claim 1, with the proviso that if m is 0 n is 0, or a salt thereof.

3. A novel compound of the formula I according to Claim 1, in which $R_1$ and $R_3$ are a substituted or unsubstituted phenyl or pyridyl group, X, Y, n, m and p have the meanings defined in Claim 1, with the proviso that if m is 0 n is 0, $R_2$ is a hydrogen atom, Alk is a lower alkylene group having 2 carbon atoms in the linear chain, or a salt thereof.

4. A novel compound of the formula I according to Claim 1, in which $R_1$ and $R_3$ are a phenyl or pyridyl group which is unsubstituted or substituted by halogen, lower alkyl, carboxyl or trifluoromethyl, n is 0 or 1, m is 0 or 1, p is 1, with the proviso that if m is 0 n is 0, $R_2$ is a hydrogen atom, Alk is a lower alkylene group having 2 carbon atoms in the linear chain, X is oxygen, and Y is the imino group or a direct bond, or a salt thereof.

5. A compound of the formula I according to Claim 1, in which $R_1$ and $R_3$ are a phenyl or 2- or 4-pyridyl group which is unsubstituted or substituted by chlorine, methyl, carboxyl or trifluoromethyl, n, m and p have the meanings defined in Claim 4, $R_2$ is a hydrogen atom, Alk is a 1,2-ethylene group, X is oxygen, and Y is the imino group, or a therapeutically applicable salt thereof.

6. 1-[2-[2-(2-Chlorophenyl)-2-imidazolin-1-yl]-ethyl]-3-(4-tolyl)-urea.

7. 1-[2-[2-(4-Pyridyl)-2-imidazolin-1-yl]-ethyl]-3-(4-tolyl)-urea.

8. 1-[2-[2-(2-Pyridyl)-2-imidazolin-1-yl]-ethyl]-3-(4-ethoxycarbonyl-phenyl)-urea.

9. 1-[2-[2-(4-Pyridyl)-2-imidazolin-1-yl]-ethyl]-3-(4-ethoxycarbonyl-phenyl)-urea.

10. 1-[2-[2-(4-Pyridyl)-2-imidazolin-1-yl]-ethyl]-3-(4-carboxyphenyl)-urea.

11. 1-[2-[2-(2-Pyridyl)-2-imidazolin-1-yl]-ethyl]-3-(4-carboxyphenyl)-urea.

12. A compound stated in any one of Claims 6 to 11 in the form of a therepeutically applicable salt thereof.

13. A pharmaceutical preparation containing any one of the compounds of Claims 1—12.

14. Any one of the compounds of Claims 1—12 for application in a process for the terapeutical treatment of the human or animal body.

15. Any one of the compounds of Claims 1—12 for use as an agent having an anti-tumour action.

16. Use of the compounds of Claims 1—12 for producing pharmaceutical preparations.

17. A process for the preparation of a novel compound of the formula I

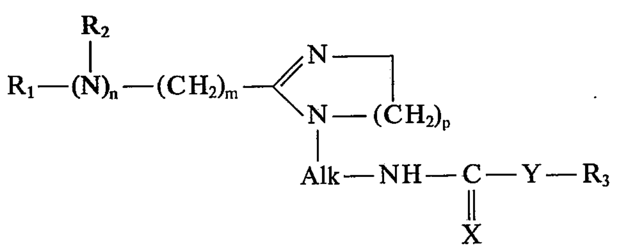

(I)

in which $R_1$ and $R_3$ are a monocyclic, carbocyclic aryl or heteroaryl, n is 0 or 1, m is 0, 1 or 2, p is 1 or 2, $R_2$ is a hydrogen atom or lower alkyl, Alk is a lower alkylene group having 2—3 carbon atoms in the linear chain, X is oxygen or sulfur, and Y is an imino group or a direct bond, or a salt thereof, which process comprises

a) reacting a compound of the general formula II

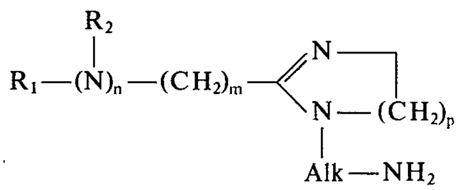

(II)

with a reactive derivative of the acid of the general formula III

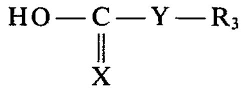

(III)

in which X, Y and $R_3$ are as defined above, or

b) reacting a compound of the general formula VI

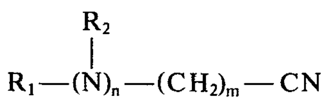

(VI)

in which $R_1$, $R_2$, n and m are as defined above, with a diethylene- or dipropylene-triamine derivative of the general formula VII

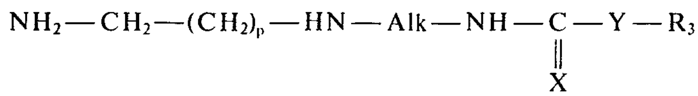

(VII)

in which p, X, Y, Alk and $R_3$ are as defined above, and, in a resulting compound of the general formula I, introducing, modifying or detaching substituents if desired, within the scope of the definition of the end products, and/or separating a resulting mixture of racemates into the pure racemates and/or resolving a resulting racemate into the optical antipodes and/or converting a resulting salt to the free compound or another salt or converting a resulting free compound to a salt thereof.

18. The compounds obtainable by the process of Claim 17.

**Revendications**

1. Urées et amides de formula générale I

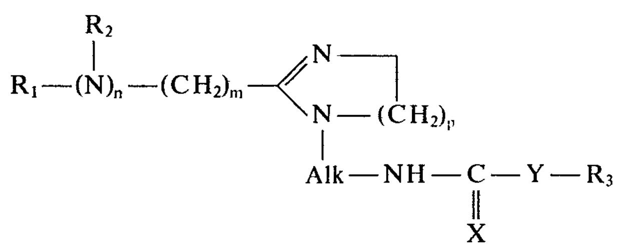

(I)

dans laquelle $R_1$ et $R_3$ représentent un reste aryle carboxylique monocyclique ou hétéroaryle, n est égal à 0 ou 1, m est égal à 0, 1 ou 2, p est égal à 1 ou 2, $R_2$ représente un atome d'hydrogène ou un groupe alkyle inférieur, Alk représente un groupe alkylène inférieur contenant 2 ou 3 atomes de carbone dans la ligne de chaîne, X représente l'oxygène ou le soufre et Y le groupe imino ou une liaison directe, et leurs sels.

2. Nouveaux composés de formule I selon la revendication 1, dans laquelle $R_1$, $R_2$, Alk, X, Y, n, m et p ont les significations indiquées dans la revendication 1, avec la restriction que lorsque m est égal à 0, n est égal à 0, et leurs sels.

3. Nouveaux composés de formule I selon la revendication 1, dans laquelle $R_1$ et $R_3$ représentent un reste phényle ou pyridyle 'eventuellement substitué, X, Y, n, m et p ont les significations indiquées dans la revendication 1, avec la restriction que lorsque m est égal à 0, n est égal à 0, $R_2$ représente un atome d'hydrogène, Alk un groupe alkylène inférieur contenant 2 atomes de carbone dans la ligne de chaîne, et leurs sels.

4. Nouveaux composés de formule I selon la revendication 1, dans laquelle $R_1$ et $R_3$ représentent un groupe phényle ou pyridyle éventuellement substitué par des halogènes, des groupes alkyles inférieurs, carboxyle ou trifluorométhyle, n est égal à 0 ou 1, m est égal à 0 ou 1, p est égal à 1, avec la

restriction que lorsque m est égal à 0, n est égal à 0, $R_2$ représente un atome d'hydrogène, Alk un groupe alkylène inférieur contenant 2 atomes de carbone dans la ligne de chaîne, X représente l'oxygène et Y le groupe imino ou une liaison directe, et leurs sels.

5. Composés de formule I selon la revendication 1, dans laquelle $R_1$ et $R_3$ représentent un groupe phényle ou pyridyle-2 ou -4 éventuellement substitué par le chlore, des groupes méthyle, carboxyle ou trifluorométhyle, n, m et p ont les significations indiquées dans la revendication 4, $R_2$ représente un atome d'hydrogène, Alk un groupe 1,2-éthylène, X l'oxygène et Y le groupe imino, et leurs sels utilisables en thérapeutique.

6. La 1-[2-[2-(2-chlorophényl)-2-imidazoline-1-yl]-éthyl]-3-(4-tolyl)-urée.

7. La 1-[2-[2-(4-pyridyl)-2-imidazoline-1-yl]-éthyl]-3-(4-tolyl)-urée.

8. La 1-[2-[2-(2-pyridyl)-2-imidazoline-1-yl]-éthyl]-3-(4-éthoxycarbonyl-phényl)-urée.

9. La 1-[2-[2-(4-pyridyl)-2-imidazoline-1-yl]-éthyl]-3-(4-éthoxycarbonyl-phényl)-urée.

10. La 1-[2-[2-(4-pyridyl)-2-imidazoline-1-yl]-éthyl]-3-(4-carboxyphényl)-urée.

11. La 1-[2-[2-(2-pyridyl)-2-imidazoline-1-yl]-éthyl]-3-(4-carboxyphényl)-urée.

12. Les composés mentionnés dans une des revendications 6 à 11, à l'état de sels utilisables en thérapeutique.

13. Compositions pharmaceutiques contenant un des composés des revendications 1 à 12.

14. Les composés des revendications 1 à 12, pour l'utilisation dans un procédé pour le traitement thérapeutique de l'organisme humain ou animal.

15. Les composés des revendications 1 à 12, pour l'utilisation en tant qu'agents anti-tumeurs actifs.

16. Utilisation des composés des revendications 1 à 12 pour la préparation de compositions pharmaceutiques.

17. Procédé de préparation des nouveaux composés de formule I

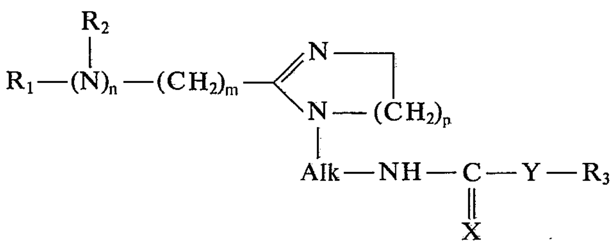

(I)

dans laquelle $R_1$ et $R_3$ représentent un reste aryle carbocyclique monocyclique ou hétéroaryle, n est égal à 0 ou 1, m est égal à 0, 1 ou 2, p est égal à 1 ou 2, $R_2$ représente un atome d'hydrogène ou un groupe alkyle inférieur, Alk représente un groupe alkylène inférieur contenant 2 à 3 atomes de carbone dans la ligne de chaîne X représente l'oxygène ou le soufre, Y le groupe imino ou une liaison directe, et de leurs sels, caractérisé en ce que:

a) on fait réagir un composé de formule générale II

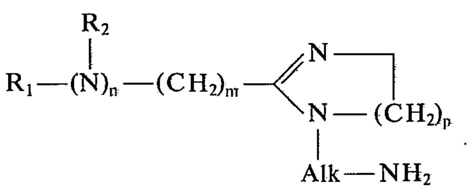

(II)

avec un dérivé réactif de l'acide de formule générale III

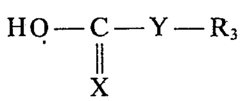

(III)

dans laquelle X, Y et $R_3$ ont les significations indiquées ci-dessus, ou bien

b) on fait réagir un composé de formule générale VI

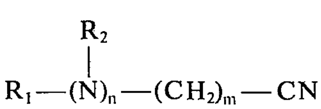

(VI)

dans laquelle $R_1$, $R_2$, n et m ont les significations indiquées ci-dessus, avec un dérivé de diéthylène- ou dipropylène-triamine respectivement, de formule générale VII

$$NH_2-CH_2-(CH_2)_p-HN-Alk-NH-\underset{\underset{\displaystyle X}{\|}}{C}-Y-R_3 \qquad (VII)$$

dans laquelle p, X, Y, Alk et $R_3$ ont les significations indiquées ci-dessus, et dans un composé obtenu, répondant à la formule générale I, si on le désire, et dans le cadre de la définition des produits finals, on introduit des substituants, on apporte des modifications et/ou on provoque des scissions, et/ou on sépare des mélanges de racémates obtenus en les racémates purs, et/ou des racémates obtenus en les antipodes optiques, et/ou on convertit des sels dobtenus en les composés libres ou en d'autres sels ou les composés libres obtenus en leurs sels.

18. Les composés obtenus par le procédé de la revendication 17.